# EUROPÄISCHE PATENTSCHRIFT

(11) **EP 4 179 061 B1**
(45) Veröffentlichungstag und Bekanntmachung des Hinweises auf die Patenterteilung: **14.08.2024**
(21) Anmeldenummer: 21749536.5
(22) Anmeldetag: 13.07.2021
(51) Int. Cl.: C02F 1/00, C02F 1/66, C12M 1/00

(54) **BIOREAKTORREINIGUNGSANLAGE MIT EINEM SÄURETANK SOWIE EINER EINRICHTUNG ZUM NEUTRALISIEREN DER SÄURE**
BIOREACTOR CLEANING SYSTEM WITH AN ACID TANK AND A DEVICE FOR NEUTRALIZING THE ACID
INSTALLATION DE NETTOYAGE DE BIORÉACTEUR COMPRENANT UN RÉSERVOIR D'ACIDE ET UN DISPOSITIF DE NEUTRALISATION DE L'ACIDE

(30) Priorität: 13.07.2020 DE 202020104033 U
(43) Veröffentlichungstag der Anmeldung: 17.05.2023
(73) Patentinhaber: Vogelsang GmbH & Co. KG, 49632 Essen (DE)
(72) Erfinder: HEINRICHS, Martin, 49632 Essen (DE)
(74) Vertreter: Eisenführ Speiser
(86) Internationale Anmeldenummer: PCT/EP2021/069425
(87) Internationale Veröffentlichungsnummer: WO 2022/013201

(56) Entgegenhaltungen:
- EP-A1- 2 098 280
- EP-A1- 2 364 893
- EP-A1- 2 789 683
- CN-A- 110 790 452
- DE-A1- 102016 115 393
- DE-A1- 19 524 960
- DE-U1- 202017 101 065
- NN / ET AL: "Bioengineering AG, CH-8636 Wald CIP-Reinigung CIP-Cleaning", 12 July 2019 (2019-07-12), https://web.archive.org/web/20190615000000*/https://bioengineering.ch/assets/Dokumente/BioCH/BioCH-Kap-17-CIP.pdf, pages 139 - 150, XP055831392, Retrieved from the Internet <URL:https://bioengineering.ch/assets/Dokumente/BioCH/BioCH-Kap-17-CIP.pdf> [retrieved on 20210810]
- SÜDOSTBAHN ET AL: "Das stille Örtchen wird aufgerüstet", 26 June 2017 (2017-06-26), St. Gallen, pages 1 - 2, XP055849569, Retrieved from the Internet <URL:https://www.sob.ch/fileadmin/images/medienmitteilungen/2017/MM_Bioreaktor-26-6-2017.pdf> [retrieved on 20211008]

## Beschreibung

Die Erfindung betrifft eine Bioreaktorreinigungsanlage zum Reinigen eines Bioreaktors, vorzugsweise eines Bioreaktors in einem Schienenfahrzeug, mit einer Absaugeinheit zum Absaugen von Flüssigkeit aus dem Bioreaktor, wobei die Absaugeinheit einen Absauganschluss zum Anschließen an den Bioreaktor aufweist, einer Zuführeinheit zum Zuführen von Flüssigkeit in den Bioreaktor, wobei die Zuführeinheit wenigstens einen ersten Zuführanschluss zum Anschließen an den Bioreaktor aufweist, einer elektronischen Steuereinheit zum Ansteuern der Absaugeinheit und der Zuführeinheit, einem Säuretank zum Aufnehmen einer wässrigen Säurelösung, einem Sammeltank zum Aufnehmen von aus dem Bioreaktor abgesaugter Flüssigkeit und einem Frischwasseranschluss zum Versorgen der Bioreaktorreinigungsanlage mit Frischwasser, wobei die Zuführeinheit mit dem Frischwasseranschluss und dem Säuretank verbunden ist, um wahlweise Frischwasser und/oder wässrige Säurelösung zu dem ersten Zuführanschluss bereitzustellen, und wobei die Absaugeinheit zum Absaugen von Flüssigkeit aus dem Bioreaktor wahlweise mit dem Säuretank oder Sammeltank verbindbar ist, um aus dem Bioreaktor abgesaugte Säurelösung dem Säuretank und aus dem Bioreaktor abgesaugtes im Wesentlichen pH-neutrales Restfluid dem Sammeltank zuzuführen. Bei stationären Anlagen kann der Sammeltank auch entfallen und die aus dem Bioreaktor abgesaugte Flüssigkeit direkt einem Kanalabfluss zugeführt werden. Die Erfindung betrifft ferner ein Verfahren zum Betreiben eine Bioreaktorreinigungsanlage sowie ein
Computerproramm.

Die Druckschrift Nn / ET AL: "Bioengineering AG, CH-8636 Wald CIP-Reinigung CIP-Cleaning", 12. Juli 2019, Seiten 139-150, offenart ein Bioreaktor-Reinigungssystem, bei dem die Produktionsanlage aufgegliedert ist in Reinigungskreisläufe, um Verunreinigungen zu entfernen und Keimfreiheit zu gewährleisten. Neben chemischen Faktoren, wie Reinigungsmittelkonzentration, -Aktivität und - temperatur, gibt es die mechanische Wirkung, die beeinflusst wird von Fließgeschwindigkeit und Turbulenz. Die Einrichtung verfügt über mehrere Sprühköpfe als auch über ein Rührwerk. Die Reinigungsgüte wird mittels Leitfähigkeitsmessung der Spülflüssigkeit kontrolliert.

Herkömmliche Bioreaktoren verfügen über einen Feststofftank mit einem Filterkorb, in den Abwasser mit festen und flüssigen Bestandteilen eingeleitet wird. Der Filterkorb trennt die festen von den flüssigen Bestandteilen. Dafür weist der Filterkorb an den begrenzenden Wänden, wie Boden- und Seitenwänden, Filterelemente auf, durch welche flüssige Elemente abfließen können und durch welche feste Elemente aufgefangen werden. Die festen Elemente sammeln sich am Boden innerhalb des Filterkorbs separiert von den flüssigen Elementen und bilden einen Filterkuchen. Die flüssigen Elemente fließen durch die Filterelemente hindurch in den Feststofftank und von dort in einen Flüssigtank, der in Fluidverbindung mit dem Feststofftank steht.

Es ist bekannt, dass sich die festen Elemente in dem Filterkorb als Filterkuchen absetzen. Zuerst bildet sich ein Filterkuchen beginnend an einer Bodenseite des Filterkorbs und anschließend an den Seiten des Filterkorbs. Dadurch wird das Wasser durch den Filterkuchen gehemmt, in den Feststofftank abzufließen. Ein Filterkuchen mit gewisser Durchlässigkeit führt zu einem effizienten Filtervorgang. Jedoch kann ein zunehmend dicker und undurchlässiger werdender Filterkuchen dazu führen, dass der Filterkorb verstopft. Dies führt zu einem ineffizienten Filterverfahren, da die Flüssigkeit kaum mehr durch den Filter gelangt. Es ist daher notwendig, den Filterkorb in regelmäßigen Abständen von Feststoffen zu reinigen, um einen ausreichenden Abfluss des Wassers in den Feststofftank zu gewährleisten.

Es ist bekannt, den Filterkuchen zu entfernen, um diesem Verstopfen zu begegnen. Oftmals wird hierbei der Filterkuchen entfernt, sobald erste Effekte einer Verstopfung auftreten. Dies hat allerdings den Nachteil, dass bereits eine ineffiziente Filterung stattgefunden hat. Es ist auch bekannt, von Zeit zu Zeit die Menge des Filterkuchens zu überprüfen, um daran festzustellen, ob eine Entfernung notwendig ist. Dies hat allerdings den Nachteil, dass die Überprüfung zufällig erfolgt, und der richtige Zeitpunkt, also weder zu früh noch zu spät, für eine Entfernung des Filterkuchens so nicht zuverlässig getroffen werden kann. Zudem ist eine Beurteilung, ob der Filterkuchen bereits so undurchlässig ist, dass er entfernt werden muss, nicht zuverlässig möglich.

Ein Problem bei solchen Reinigungsvorgängen liegt jedoch darin, dass Bioreaktoranlagen in der Regel als geschlossenes System aufgebaut sind und daher eine Ermittlung des Verschmutzungsgrades und der Ursache für eine unzureichende Filterung nur sehr aufwendig möglich ist. Gerade schon bestehende Bioreaktoren verfügen häufig nicht über Schnittstellen, mittels derer für die Bestimmung einer Fehlerursache oder eines Verschmutzungsgrades notwendige Informationen oder auch nur dafür hilfreiche Daten ausgelesen werden können. Dies ist insbesondere dann zusätzlich erschwert, wenn ein solcher Bioreaktor an Bord eines Fahrzeugs, wie beispielsweise eines gleisgebundenen Waggons, eingebaut ist, um das dort anfallende Schmutzwasser zu reinigen. In solchen Anwendungsfällen ist eine Wartung und Sicherstellung der Funktion des Bioreaktors häufig dezentral und ohne dessen Ausbau gewünscht, zugleich aber aufgrund der notwendigen Kompaktheit der Zugang zum Bioreaktor und zu Daten, die dessen Zustand beschreiben, nicht oder nur sehr aufwendig möglich.

Ferner besteht ein Problem darin, die eingesetzte Säure beim Reinigen zu rezyklieren bzw. zu entsorgen oder zu neutralisieren. Üblicherweise wird Säure dem Bioreaktor zugeführt, um Kalkablagerungen oder dergleichen zu entfernen. Dies soll nicht nur im Bioreaktor selbst, sondern auch in den Leitungen geschehen. Die Säure kann allerdings im Anschluss nicht ohne Weiteres entsorgt werden, sondern muss zuvor neutralisiert werden. Dies ist einerseits zeitaufwendig, andererseits besteht ein Problem darin, den pH-Wert korrekt einzustellen, um die so neutralisierte Säure dann entsorgen zu können.

Aufgabe der Erfindung ist es, eine Bioreaktorreinigungsanlage, ein Verfahren und Computerprogramm der eingangs genannten Art anzugeben, welche einen effizienteren Einsatz der Säure erlauben.

Diese Aufgabe wird bei einer Bioreaktorreinigungsanlage der eingangs genannten Art dadurch gelöst, dass diese eine Dosiereinheit aufweist, die eine oder mehrere Säurekanister-Anschlüsse zum Anschließen von Säurekanistern und einen oder mehrere Basekanister-Anschlüsse zum Anschließen von Basekanistern aufweist, einen Säure-Dosierer, der eingangsseitig mit dem einen oder den mehreren Säurekanister-Anschlüssen und ausgangsseitig mit dem Säuretank und/oder dem Bioreaktor verbindbar ist, und einen Base-Dosierer aufweist, der eingangsseitig mit dem einen oder den mehreren Basekanister-Anschlüssen und ausgangsseitig mit dem Säuretank und/oder dem Bioreaktor verbindbar ist, sowie einen Mischer aufweist, wobei der Mischer eingangsseitig mit einem Frischwasseranschluss und dem Säure-Dosierer und/oder Base-Dosierer und ausgangsseitig mit dem Säuretank und/oder dem Bioreaktor verbindbar ist, um diesem eine zum Neutralisieren der in dem Säuretank und/oder Bioreaktor vorhandenen Säurelösung nutzbare Lösung zuzuführen.

Der Erfindung liegt die Erkenntnis zugrunde, dass durch den Einsatz des Mischers, der mit dem Säure-Dosierer und/oder Base-Dosierer verbunden ist, eine Flüssigkeit bereitgestellt werden kann, die einerseits in dem Säuretank direkt als wässrige Säurelösung nutzbar ist, andererseits aber sowohl ein Nachdosieren von Säure ermöglicht als auch ein Neutralisieren durch die gezielte Zugabe von Base. Hierdurch ist es nicht mehr erforderlich, die in dem Säuretank befindliche Säure, die beispielsweise nach einem Reinigungsvorgang aus dem Bioreaktor abgesaugt wurde, zunächst zu sammeln, dann zu einer Station zu verbringen, wo die aus dem Bioreaktor abgesaugte Säurelösung dann durch Zugabe von Base neutralisiert wird. Vielmehr wird die verbrauchte Säure direkt in der Bioreaktorreinigungsanlage neutralisiert und kann sodann entsorgt werden. Die Dosiereinheit kann aber auch über entsprechende Leitungen und Ventile direkt mit dem Bioreaktor verbunden werden, beispielsweise über die Zuführeinheit. Auf diese Weise kann zum Reinigen nutzbare saure Flüssigkeit unmittelbar von der Dosiereinheit in den Bioreaktor gegeben werden. Zudem ist es möglich, nach Abschluss der Reinigung, die in dem Bioreaktor vorhandene wässrige Säurelösung durch entsprechende Zugabe von Base mittels der Dosiereinheit in dem Bioreaktor selbst zu neutralisieren. Hierdurch kann Frischwasser gespart werden, welches sonst zum Spülen des Bioreaktors nach dem Reinigen des Bioreaktors mit wässriger Säurelösung genutzt werden müsste.

Vorzugsweise weist die Bioreaktorreinigungsanlage einen ersten pH-Sensor zum Erfassen eines ersten pH-Werts einer dem Säuretank zugeführten Flüssigkeit und zum Bereitstellen eines ersten pH-Signals an der elektronischen Steuereinheit auf. Der erste pH-Sensor ist vorzugsweise stromabwärts des Mischers angeordnet und stromaufwärts von einem Einlass des Säuretanks. Auf diese Weise kann der erste pH-Sensor den pH-Wert der im Säuretank befindlichen Flüssigkeit erfassen. Andere Einlässe als den Einlass von dem ersten pH-Sensor aus in den Säuretank sind vorzugsweise nicht vorgesehen.

Weiterhin ist bevorzugt, dass ein zweiter pH-Sensor zum Erfassen eines zweiten pH-Werts einer über den Absauganschluss abgesaugten Flüssigkeit vorgesehen ist. Der zweite pH-Sensor kann auch zusätzlich oder alternativ stromaufwärts des Mischers angeordnet sein. Vorzugsweise wird Flüssigkeit, die aus dem Bioreaktor über den Absauganschluss abgesaugt wurde, so durch die Bioreaktorreinigungsanlage geleitet, dass der zweite pH-Sensor und/oder der erste pH-Sensor den pH-Wert dieser Flüssigkeit erfassen kann. Vorzugsweise wird die Flüssigkeit so von dem Absauganschluss innerhalb der Bioreaktorreinigungsanlage geleitet, dass sie zunächst dem zweiten pH-Sensor zugeführt wird, dann dem Mischer, dann dem ersten pH-Sensor und dann in den Säuretank gelangt. Die Bioreaktorreinigungsanlage weist im Inneren vorzugsweise eine entsprechende Verrohrung und entsprechende Ventile auf, wobei die Ventile teilweise oder vollständig von der elektronischen Steuereinheit gesteuert werden können.

In einer bevorzugten Weiterbildung weist der Säure-Dosierer einen Säure-Ejektor auf, der eingangsseitig mit dem Frischwasseranschluss verbindbar ist. Der Säure-Ejektor weist somit zwei Eingangsanschlüsse auf, nämlich einen, der mit dem einen oder den mehreren Säurekanister-Anschlüssen verbindbar oder verbunden ist und einen, der mit dem Frischwasseranschluss verbindbar oder verbunden ist. In dem Säure-Ejektor werden dann Säure aus dem wenigsten einen Säurekanister sowie Frischwasser gemischt und dann dem Mischer bereitgestellt.

In übereinstimmender Weise weist der Base-Dosierer vorzugsweise einen Base-Ejektor auf, der eingangsseitig mit dem Frischwasseranschluss verbindbar ist. Auch der Base-E-jektorweist daher zwei Eingänge auf, nämlich einen, der mit dem einen oder den mehreren Basekanister-Anschlüssen verbunden oder verbindbar ist sowie einen, der mit dem Frischwasseranschluss verbindbar oder verbunden ist. In dem Base-Ejektor werden dann Base und Frischwasser gemischt und dem Mischer zugeführt. Auf diese Weise lässt sich eine besonders einfache Dosierung von Säure und Base erreichen. Vorzugsweise sind den Säure- bzw. Base-Ejektoren jeweils Ventile vor- und nachgeschaltet, um eine Einmischung von Säure und Base in Frischwasser noch besser steuern zu können. Diese vor- und nachgeschalteten Ventile sind vorzugsweise wiederum mit der elektronischen Steuereinheit verbunden, sodass diese von der elektronischen Steuereinheit gesteuert werden können. Vorzugsweise weist darüber hinaus der Mischer eine Frischwasserdrossel auf, zum Drosseln des dem Mischer zugeführten Frischwassers. Hierdurch kann weiter die Dosierung von Säure, Base und Frischwasser noch besser gesteuert werden. Auch die Drossel ist vorzugsweise mit der elektronischen Steuereinheit verbunden, sodass diese von der elektronischen Steuereinheit gesteuert und eingestellt werden kann.

In einer weiteren bevorzugten Ausführungsform weist die Bioreaktorreinigungsanlage einen ersten Durchflussmesser zum Messen des am Mischer und/oder dem Säure-Dosierer und/oder dem Base-Dosierer zugeführten Frischwassers auf. Der erste Durchflussmesser kann stromaufwärts des Mischers und stromabwärts eines Frischwasseranschlusses angeordnet sein. Um eine richtige Dosierung von Säure, Base und Frischwasser für den Säuretank bereitzustellen, ist es vorteilhaft, die Flussrate des Frischwassers zu kennen. Der Durchflussmesser stellt vorzugsweise ein Volumenstromsignal an der elektronischen Steuereinheit bereit, sodass diese in Abhängigkeit davon und in Abhängigkeit von einem gewünschten pH-Wert die Eindosierung von Säure und Base steuern kann.

Vorzugsweise ist ferner ein zweiter Durchflussmesser zum Messen der dem Säure-Dosierer zugeführten Säure vorgesehen. Auch hier ist es vorteilhaft, den Durchfluss zu kennen. Dies ist besonders dann relevant, wenn der Säuretank beispielsweise zu Beginn oder vor einer Reinigung zunächst mit einer wässrigen Säurelösung gefüllt werden soll und insofern nur Säure und Frischwasser gemischt werden. Zwar ist es auch möglich, den pH-Wert im Säuretank zu messen, allerdings ist durch den Durchflussmesser eine bereits vor der Mischung liegende Flussrate erfassbar, sodass basierend auf den Volumenströmen von Säure und Frischwasser eine Mischung erzeugt werden kann.

In einer weiteren bevorzugten Ausführungsform sind die Absaugeinheit und/oder die Zuführeinheit betreibbar zum Pumpen von Flüssigkeit aus dem Säuretank heraus zu dem Mischer und zurück in den Säuretank. Auf diese Weise kann der Flüssigkeit, die in dem Säuretank vorhanden ist, weitere Säure und/oder Base zugemischt werden. Würde Säure oder Base direkt in den Säuretank gegeben, müsste sie dort vermischt werden. Da dies nicht ohne Weiteres möglich ist, herrscht in dem Säuretank im Stand der Technik in der Regel ein Konzentrationsgefälle. Durch das zyklische Pumpen von dem Säuretank zu dem Mischer und zurück in den Säuretank ist es möglich, eine sehr homogene Flüssigkeit in dem Säuretank zu erzielen, die vorteilhaft für die Reinigung des Bioreaktors eingesetzt werden kann. Auch ist es einfacher möglich, auf diese Weise die in dem Säuretank befindliche Säure zu neutralisieren. Im Stand der Technik besteht dahingegen das Problem, dass zwar zu der im Säuretank befindlichen Säure ausreichend Base hinzugegeben wird, aufgrund des Konzentrationsgefälles dann aber beim Entsorgen punktuell und/oder abschnittsweise Grenzwerte überschritten werden, wodurch Leitungen beschädigt und Umweltvorschriften nicht eingehalten werden können. Durch das Zuführen der in dem Säuretank vorhandenen Flüssigkeit zu dem Mischer und von dort aus zurück in den Säuretank wird dieses Problem behoben und eine homogene Flüssigkeit kann erreicht werden.

Alternativ oder zusätzlich, sind die Absaugeinheit und/oder die Zuführeinheit betreibbar zum Pumpen von Flüssigkeit aus dem Bioreaktor heraus zu dem Mischer und zurück in den Bioreaktor. Hierdurch kann die wässrige Säurelösung nach der Reinigung des Bioreaktors auch in diesem selbst neutralisiert werden. Hierzu wird die Flüssigkeit aus dem Bioreaktor abgesaugt, vorzugsweise mittels der Absaugeinheit, dann zu der Dosiereinheit befördert, in welcher dann Base zudosiert wird, und anschließend zurück in den Bioreaktor, vorzugsweise mittels der Zuführeinheit. Dieser Zyklus kann wiederholt werden, bis ein gewünschter, neutraler pH-Wert erreicht ist. Sollte zwischenzeitlich zu viel Base zudosiert worden sein, muss gegebenenfalls Säure zudosiert werden, um wieder etwas aufzusäuern.

Zusätzlich oder alternativ sind die Absaugeinheit und/oder die Zuführeinheit vorzugsweise zum Pumpen von Flüssigkeit aus dem Säuretank und/oder dem Bioreaktor heraus zu dem ersten oder zweiten pH-Sensor und zurück zum Säuretank und/oder Bioreaktor betreibbar. Auf diese Weise lässt sich der pH-Wert der im Säuretank bzw. im Bioreaktor befindlichen Flüssigkeit einfach bestimmen. Ist der zweite pH-Sensor stromaufwärts des Mischers angeordnet, wird die Flüssigkeit zunächst zum zweiten pH-Sensor, dann zum Mischer, dann zum ersten pH-Sensor und zurück zum Säuretank geleitet. Das heißt, an dem zweiten pH-Sensor kann zunächst der pH-Wert der aus dem Säuretank abgesaugten Flüssigkeit ermittelt werden und dann bedarfsweise Säure bzw. Base in dem Mischer zugeführt werden. An dem ersten pH-Sensor kann dann diese Mischung kontrolliert werden, dem Säuretank wieder zugeführt werden und so fort, bis ein entsprechender pH-Wert am zweiten pH-Sensor, also am Eingang des Mischers, erfasst wird. Vorzugsweise ist am Eingang und am Ausgang des Mischers der pH-Wert gleich, wenn der Ziel-pH-Wert erreicht wurde.

In einer weiteren bevorzugten Ausführungsform ist die elektronische Steuereinheit ausgebildet zum Ermitteln eines eine Qualität von in dem Säuretank vorhandener wässriger Säurelösung angebenden Säurequalitätswerts, Vergleichen des ermittelten Säurequalitätswerts mit einem vorbestimmten Säurevergleichswert und in Abhängigkeit von dem Vergleichen: Einleiten einer Neutralisation der wässrigen Säurelösung in dem Säuretank. Ein solcher Säurequalitätswert kann beispielsweise eine Beladung mit Feststoffen, ein Verschmutzungsgrad oder dergleichen sein.

Besonders bevorzugt ist der Säurequalitätswert der erste pH-Wert und die elektronische Steuereinheit ist ausgebildet zum Ermitteln des ersten pH-Werts von in dem Säuretank vorhandener wässriger Säurelösung, Vergleichen des ermittelten ersten pH-Werts mit einem vorbestimmten pH-Schwellwert und für den Fall, dass der ermittelte erste pH-Wert den pH-Schwellwert überschreitet: Einleiten einer Neutralisation der wässrigen Säurelösung in dem Säuretank. Die wässrige Säurelösung, die im Säuretank vorhanden ist, wird vorzugsweise für wenigstens einen Reinigungszyklus eines Bioreaktors verwendet. Bei einem Reinigungszyklus wird die wässrige Säurelösung über den Zuführanschluss dem Bioreaktor zugeführt, wird innerhalb des Bioreaktors durch verschiedene Leitungen und/oder Siebe oder dergleichen geführt, und dann wird die so genutzte und dabei teilweise oder vollständig verbrauchte Säure über den Absauganschluss in die Bioreaktorreinigungsanlage abgesaugt und dort wiederum dem Säuretank zugeführt. Es hat sich gezeigt, dass mit dieser wässrigen Säurelösung auch zwei oder mehr Reinigungsvorgänge durchgeführt werden können, allerdings nicht beliebig viele. Nach einer gewissen Anzahl Reinigungsvorgängen, abhängig von dem Verschmutzungsgrad des Bioreaktors, ist die Säurelösung endgültig verbraucht. Dies wird vorzugsweise dadurch ermittelt, dass der Säurequalitätswert bzw. vorzugsweise der erste pH-Wert, den pH-Schwellwert überschreitet.

Die elektronische Steuereinheit ist vorzugsweise dazu ausgebildet, den Säurevergleichswert und/oder den vorbestimmten pH-Schwellwert aus einer Datenbank zu ermitteln und zu laden. Der Säurevergleichswert bzw. der vorbestimmte pH-Schwellwert können abhängig sein von der Art des Bioreaktors, von der Art der verwendeten Säure oder anderen Parametern. In Abhängigkeit von diesen Parametern ermittelt die elektronische Steuereinheit dann die entsprechenden Werte und lädt diese aus einer Datenbank, oder aus einem entfernt angeordneten Speicher, beispielsweise über das Internet, oder von einem Cloud-Dienst.

In einer weiteren bevorzugten Ausführungsform ist die elektronische Steuereinheit dazu ausgebildet, zum Neutralisieren der in dem Säuretank vorhandenen wässrigen Säurelösung, während die Absaugeinheit und/oder die Zuführeinheit die Flüssigkeit aus dem Säuretank heraus zu dem ersten oder zweiten pH-Sensor und zurück in den Säuretank pumpt, den Base-Dosierer zu veranlassen, Base in den Mischer auszugeben, bis in dem Säuretank ein neutraler Ziel-pH-Wert erreicht ist. Auf diese Weise kann eine Durchlaufneutralisation erreicht werden, in der nur so viel Base verwendet wird wie erforderlich. Zudem wird stets eine homogene Flüssigkeit erreicht, sodass weder zu viel noch zu wenig Base zugeführt wird und so eine effiziente Neutralisation der Base erreicht wird.

In einem zweiten Aspekt löst die Erfindung die eingangs genannte Aufgabe durch ein Verfahren zum Betreiben einer Bioreaktorreinigungsanlage, vorzugsweise einer Bioreaktorreinigungsanlage nach einer der vorstehend beschriebenen bevorzugten Ausführungsformen einer Bioreaktorreinigungsanlage gemäß dem ersten Aspekt der Erfindung, wobei das Verfahren die Schritte aufweist: Ermitteln eines eine Qualität von in einem Säuretank der Bioreaktorreinigungsanlage vorhandener wässriger Säurelösung angebenden Säurequalitätswerts, Vergleichen des ermittelten Säurequalitätswerts mit einem vorbestimmten Säurevergleichswert, und in Abhängigkeit von dem Vergleichen: Einleiten einer Neutralisation der wässrigen Säurelösung in dem Säuretank.

Dem Verfahren liegt die Idee zugrunde, dass in Abhängigkeit von der Qualität der Säurelösung in dem Säuretank ein Neutralisationsverfahren durchgeführt werden soll. Die in dem Säuretank vorhandene wässrige Säurelösung wird wenigstens einmal zum Reinigen des Bioreaktors genutzt. Mit jedem Mal der Nutzung der wässrigen Säurelösung in dem Säuretank durch Zuführen dieser zum Bioreaktor, anschließendem Absaugen aus dem Bioreaktor und Rückführen in den Säuretank kann sich die Qualität der wässrigen Säurelösung verschlechtern. Demnach wird gemäß dem Verfahren vorzugsweise ein Vergleich eines Säurequalitätswerts mit einem vorbestimmten Säurevergleichswert durchgeführt. In Abhängigkeit von diesem Vergleich, insbesondere wenn der Säurequalitätswert den vorbestimmten Säurevergleichswert unterschreitet, wird eine Neutralisation der wässrigen Säurelösung eingeleitet. Dies ist erforderlich, um die wässrige Säurelösung nach der Neutralisation entsorgen zu können. Die Neutralisation ist vorzugsweise eine Durchlaufneutralisation.

Besonders bevorzugt ist der Säurequalitätswert ein erster pH-Wert. Der pH-Wert der wässrigen Säurelösung eignet sich besonders als Säurequalitätswert, da über diesen eine Aussage über die Fähigkeit der wässrigen Säurelösung, Verkalkungen in dem Bioreaktor zu lösen, getroffen werden kann. In diesem Fall umfasst das Verfahren dann vorzugsweise die Schritte: Ermitteln des ersten pH-Werts von in dem Säuretank vorhandener wässriger Säurelösung, und Vergleichen des ermittelten ersten pH-Werts mit einem vorbestimmten pH-Schwellwert, der dann in diesem Fall der Säurevergleichswert ist. Falls der ermittelte erste pH-Wert den pH-Schwellwert überschreitet, die wässrige Säurelösung also nicht mehr sauer genug ist, wir die Neutralisation der wässrigen Säurelösung eingeleitet.

Weiterhin ist bevorzugt, dass das Verfahren umfasst: Ermitteln und Laden des Säurevergleichswerts und/oder des vorbestimmten pH-Schwellwerts aus einer Datenbank. Die Datenbank kann intern innerhalb der Bioreaktorreinigungsanlage vorgesehen sein oder über ein entferntes System, beispielsweise das Internet oder einen Cloud-Dienst übermittelt werden. Der Säurevergleichswert bzw. der vorbestimmte pH-Schwellwert kann abhängig sein von Parametern, die ein Benutzer in die Vorrichtung eingibt oder von Werten, die mittels eines oder mehrerer Sensoren über die wässrige Säurelösung erfasst werden.

Das Verfahren umfasst vorzugsweise ferner die Schritte: Pumpen von Flüssigkeit aus dem Säuretank heraus zu einem Mischer der Bioreaktorreinigungsanlage und zurück in den Säuretank. Vorzugsweise umfasst das Verfahren ferner während des Pumpens von Flüssigkeit aus dem Säuretank heraus zu einem Mischer der Bioreaktorreinigungsanlage und zurück in den Säuretank: Neutralisieren der Flüssigkeit. Vorzugsweise umfasst das Verfahren ferner: Einmischen in dem Mischer von Säure und/oder Base und/oder Frischwasser. Durch Einmischen von Säure, Base oder Frischwasser kann die Flüssigkeit neutralisiert werden bzw. deren pH-Wert weiter eingestellt werden. Dies erfolgt vorzugsweise im Mischer und nicht im Säuretank direkt. Auf diese Weise ist eine Durchlaufneutralisation bzw. Durchlaufsäuerung möglich, um den pH-Wert der wässrigen Säurelösung in dem Säuretank zielgenau einstellen zu können.

Vorzugsweise wird ferner ein erster pH-Wert stromabwärts des Mischers erfasst. Ein zweiter pH-Wert wird vorzugsweise stromaufwärts des Mischers erfasst. Durch Erfassen der ersten und zweiten pH-Werte ist es möglich, die Einmischung von Säure, Base oder Frischwasser in den Flüssigkeitsstrom in dem Mischer zu regeln. Aus diesem Grund umfasst das Verfahren vorzugsweise ferner den Schritt: in Abhängigkeit von dem ersten und/oder zweiten pH-Wert, Einmischen von Säure und/oder Base in dem Mischer zum Erzielen eines neutralen Ziel-pH-Werts in dem Säuretank. Der neutrale Ziel-pH-Wert liegt vorzugsweise in einem Bereich von 6,5 - 10. Bevorzugt ist auch ein Bereich von 6,5 - 9, 7 - 9 oder insbesondere 7 - 8. Anstelle des neutralen Ziel-pH-Werts kann auch jeder andere pH-Wert eingestellt werden. Im Grunde reicht es aus, einen pH-Wert, nämlich den ersten oder zweiten pH-Wert zu erfassen, um auf diese Weise einen geschlossenen Regelkreis zu bilden. Das Erfassen von zwei pH-Werten, stromaufwärts und stromabwärts des Mischers, ermöglicht allerdings eine genauere Regelung und ermöglicht zudem, Flüssigkeit an dem Mischer vorbei, direkt zum ersten pH-Sensor zu leiten, beispielsweise, wenn das Einmischen von Säure, Base oder Frischwasser nicht gewünscht ist. Beispielsweise kann es denkbar und bevorzugt sein, aus dem Bioreaktor abgesaugte Flüssigkeit nur dem ersten pH-Sensor zuzuführen, ohne ein Durchleiten durch den Mischer und den zweiten pH-Sensor zu nutzen.

Das Verfahren umfasst vorzugsweise ferner den Schritt: Wenn der neutrale Ziel-pH-Wert erreicht ist: Pumpen der Flüssigkeit aus dem Säuretank in einen Sammeltank der Bioreaktorreinigungsanlage und/oder einen Entsorgungskanal. In dem Sammeltank der Bioreaktorreinigungsanlage können weitere Flüssigkeiten, wie beispielsweise aus dem Bioreaktor abgesaugte Restflüssigkeit oder Flüssigkeit, die in einem mechanischen Reinigungsprozess verwendet wurde, gesammelt werden. Aus dem Sammeltank heraus kann dann die dort befindliche Flüssigkeit entsorgt werden. Der Säuretank ist üblicherweise deutlich kleiner ausgebildet als der Sammeltank, sodass es bevorzugt ist, die neutralisierte Flüssigkeit aus dem Säuretank in den Sammeltank zu bringen. Der Säuretank ist dann leer und es kann eine neue wässrige Säurelösung angemischt werden, um beispielsweise einen weiteren Reinigungsprozess eines Bioreaktors durchzuführen, ohne den Sammeltank zunächst entleeren zu müssen. Ist die Bioreaktorreinigungsanlage nicht als mobile sondern als stationäre Anlage ausgebildet, ist es nicht erforderlich, die neutralisierte Flüssigkeit in dem Sammeltank zwischenzuspeichern; sie kann in diesem Fall direkt in einen Entsorgungskanal geleitet werden.

In dem Verfahren kann ferner vorgesehen sein, dass in Abhängigkeit von dem Vergleichen des ermittelten Säurequalitätswerts mit dem vorbestimmten Säurevergleichswert die Schritte ausgeführt werden: Zuführen von Flüssigkeit aus dem Säuretank zu dem Bioreaktor zum Reinigen des Bioreaktors, Absaugen von Flüssigkeit aus dem Bioreaktor und Zuführen der abgesaugten Flüssigkeit zu dem Säuretank. Diese Schritte werden vorzugsweise dann ausgeführt, wenn der ermittelte Säurequalitätswert den Säurevergleichswert überschreitet bzw. ermittelt wird, dass die Qualität der wässrigen Säurelösung in dem Säuretank ausreichend ist.

Um die Reinigungswirkung derwässrigen Säurelösung zu verbessern, kann das Verfahren ferner den Schritt aufweisen: Einperlen von Luftblasen in die Flüssigkeit beim Zuführen der Flüssigkeit zu dem Bioreaktor. Auf diese Weise wird der wässrigen Säurelösung, die dem Bioreaktor als Flüssigkeit zugeführt wird, Sauerstoff hinzugefügt, wodurch eine chemische Reaktion zwischen der wässrigen Säurelösung und Kalk in dem Bioreaktor verbessert werden kann. Zusätzlich kann auch bei einem Zirkulieren von Flüssigkeit und/oder wässriger Säurelösung durch Leitungen der Bioreaktorreinigungsanlage und/oder des Bioreaktors Luft eingeperlt werden Hierdurch kann zusätzlich durch die Luftblasen eine mechanische Reinigung der Leitungen erreicht werden.

In einem dritten Aspekt löst die Erfindung die eingangs genannte Aufgabe durch ein Computerprogrammprodukt, umfassend Codemittel, die, wenn auf einem Computer ausgeführt, eine Bioreaktorreinigungsanlage veranlassen, die Schritte gemäß dem Verfahren nach einem der vorstehend bevorzugten Ausführungsformen eines Verfahrens gemäß dem zweiten Aspekt der Erfindung auszuführen. Der Computer ist vorzugsweise Teil der Bioreaktorreinigungsanlage. Das Computerprogrammprodukt wird vorzugsweise auf einem Speicher, wie insbesondere einem optischen Speichermittel, oder als Download bereitgestellt.

Ausführungsformen der Erfindung werden nun nachfolgend anhand der Zeichnungen beschrieben. Diese sollen die Ausführungsformen nicht notwendigerweise maßstäblich darstellen, vielmehr sind die Zeichnungen, wenn dies zur Erläuterung dienlich ist, in schematisierter und/oder leicht verzerrter Form ausgeführt. Im Hinblick auf Ergänzungen der aus den Zeichnungen unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in den Zeichnungen sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, den Zeichnungen und/oder den Ansprüchen offenbarten Merkmale. Die allgemeine Idee der Erfindung ist nicht beschränkt auf die exakte Form oder das Detail der im Folgenden gezeigten und beschriebenen bevorzugten Ausführungsformen oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein. Der Einfachheit halber sind nachfolgend für identische oder ähnliche Teile oder Teile mit identischer oder ähnlicher Funktion gleiche Bezugszeichen verwendet.

Weitere Vorteile, Merkmale und Einzelheiten der Erfindung ergeben sich aus der nachfolgenden Beschreibung der bevorzugten Ausführungsformen sowie anhand der Zeichnungen; diese zeigen in:
- Figur 1: eine schematische Darstellung der Bioreaktorreinigungsanlage im Zusammenhang mit einem Bioreaktor sowie weiteren Elementen;
- Figur 2: eine schematische Seitenansicht der Bioreaktorreinigungsanlage, teilweise freigeschnitten;
- Figur 3a - 3d: vier Darstellungen von Säurekanistern;
- Figur 4: einen Schaltplan der Bioreaktorreinigungsanlage;
- Figur 5: einen Querschnitt eines Ejektors;
- Figur 6: einen schematischen Schaltplan eines Mischers mit weiteren Elementen; und in
- Figur 7: ein Diagramm der Neutralisation der wässrigen Säurelösung.

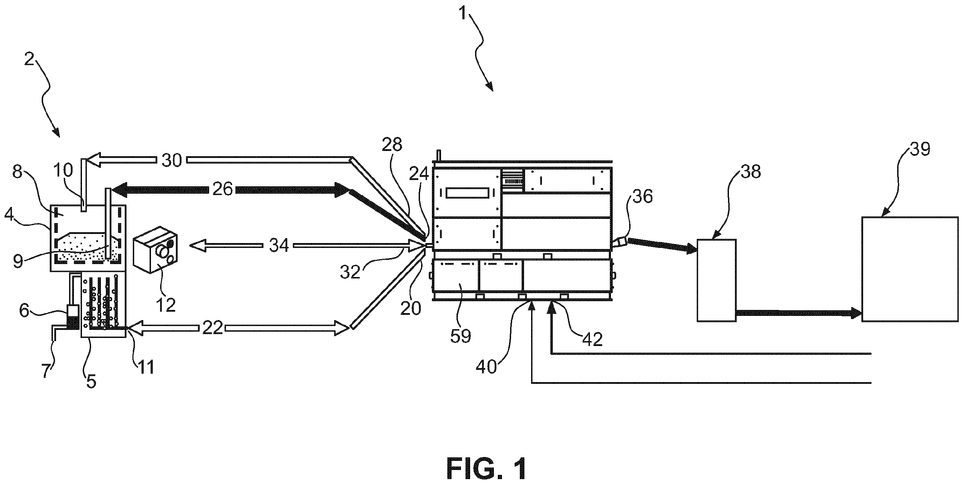

Eine Bioreaktorreinigungsanlage 1 kann als mobile Bioreaktorreinigungsanlage ausgebildet sein, wie in Fig. 1 gezeigt, oder auch als stationäre Bioreaktorreinigungsanlage. Eine mobile Bioreaktorreinigungsanlage kann typischerweise zu einem Zug, in dem ein Bioreaktor 2 angeordnet ist, verbracht werden. Bioreaktor 2 in Zügen sind im Allgemeinen bekannt und werden hier nicht weiter im Detail beschrieben. In Figur 1 ist beispielhaft ein vertikal ausgerichteter Bioreaktor 2 gezeigt, mit einem Feststofftank 4, einem Flüssigkeitstank 5 sowie einem Hygienisierer 6, der einen Auslass 7 aufweist, zum Ablassen von Flüssigkeit. In dem Feststofftank 4 ist ein Filterkorb 8 vorgesehen, in den sowohl ein 2-Zoll-Schlauch 9 bodennah endet, als auch eine Reinigungsdüse 10, um dem Feststofftank 4 Wasser unter Hochdruck zuzuführen, um einen in dem Filterkorb 8 aufgebauten Filterkuchen abzureinigen. An dem Flüssigkeitstank 5 ist ferner ein 1-Zoll-Anschluss 11 ausgebildet, um Flüssigkeit aus dem Flüssigkeitstank 5 abzusaugen oder diesem zuzuführen. Ferner umfasst der Bioreaktor 2 eine Steuerung 12, die beispielsweise Sensoren des Bioreaktors 2 auslesen kann.

Die Bioreaktorreinigungsanlage 1 weist Anschlüsse auf, über die diese mit dem Bioreaktor 2 verbindbar ist. Um beispielsweise Flüssigkeit aus dem Bioreaktor 2 abzusaugen, weist die Bioreaktorreinigungsanlage 1 einen Absauganschluss 20 auf, der über eine Absaugleitung 22 mit dem 1-Zoll-Anschluss 11 des Flüssigkeitstanks 5 des Bioreaktors 2 verbindbar ist. Ferner weist die Bioreaktorreinigungsanlage 1 einen Zuführanschluss 24 auf, der über eine Zuführleitung 26 mit dem 2-Zoll-Schlauch 9 des Bioreaktors 2 verbindbar ist, um über diesen, Flüssigkeit in den Bioreaktor 2, genauer gesagt den Feststofftank 4, zuzuführen. Die Bioreaktorreinigungsanlage 1 weist darüber hinaus einen Hochdruckanschluss 28 auf, der über einen Hochdruckschlauch 30 mit der Reinigungsdüse 10 verbindbar ist, sowie einen elektronischen Steueranschluss 32, der über eine Signalleitung 34 mit der Steuerung 12 des Bioreaktors 2 verbindbar ist.

Die Bioreaktorreinigungsanlage 1 weist ferner einen Entsorgungsanschluss 36 auf, über den die Bioreaktorreinigungsanlage 1 mit einem externen Tank 38 verbindbar ist, der mit einer externen Vakuumquelle 39 verbunden ist, um so Flüssigkeit aus der Bioreaktorreinigungsanlage 1 abzusaugen. Eingangsseitig weist die Bioreaktorreinigungsanlage 1 einen Spannungsanschluss 40 sowie einen Frischwasseranschluss 42 auf.

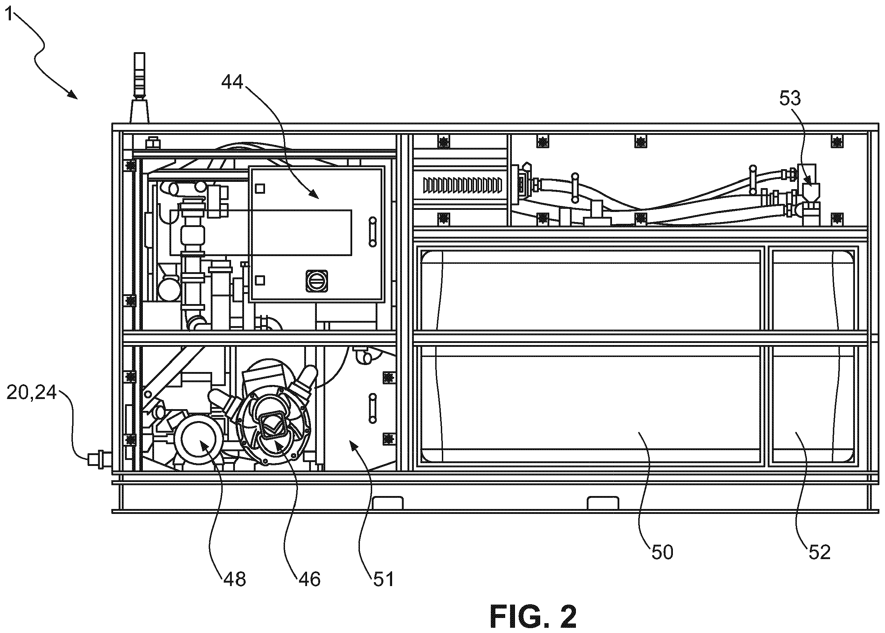

Im Inneren der Bioreaktorreinigungsanlage 1 (Figur 2) weist diese eine elektronische Steuereinheit 44 auf, die einen Speicher mit Programmcode sowie einen Prozessor zum Ausführen des Programmcodes aufweist. Die elektronische Steuereinheit 44 steuert verschiedene Funktionen der Bioreaktorreinigungsanlage 1, wie sich insbesondere aus der weiteren Beschreibung ergibt. Beispielsweise steuert die elektronische Steuereinheit 44 eine Pumpe 46 sowie eine Hochdruckpumpe 48. Die Pumpe 46 kann sowohl dazu verwendet werden, ein Vakuum an dem Absauganschluss 20 bereitzustellen, als auch Flüssigkeit zu dem Zuführanschluss 24 zu pumpen. Die Hochdruckpumpe 48 dient dazu, eine Flüssigkeit mit Hochdruck an dem Hochdruckanschluss 28 bereitzustellen. Ferner sind im Inneren der Bioreaktorreinigungsanlage 1 ein Sammeltank 50 und ein Säuretank 52 vorgesehen, wobei fürden Sammeltank50 ein erster Füllstandssensor51 und fürden Säuretank52 ein zweiter Füllstandssensor 53 vorgesehen sind.

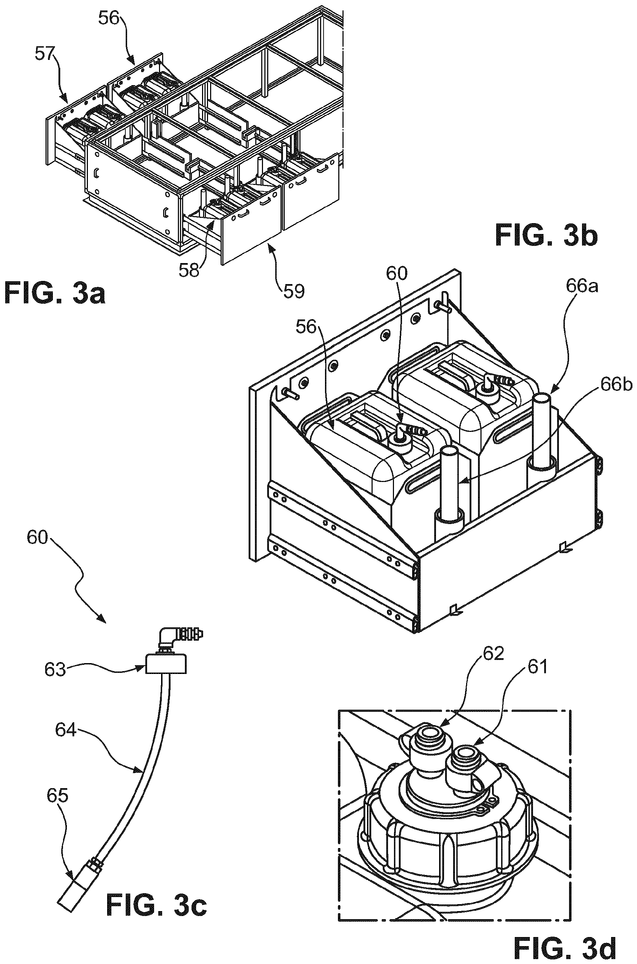

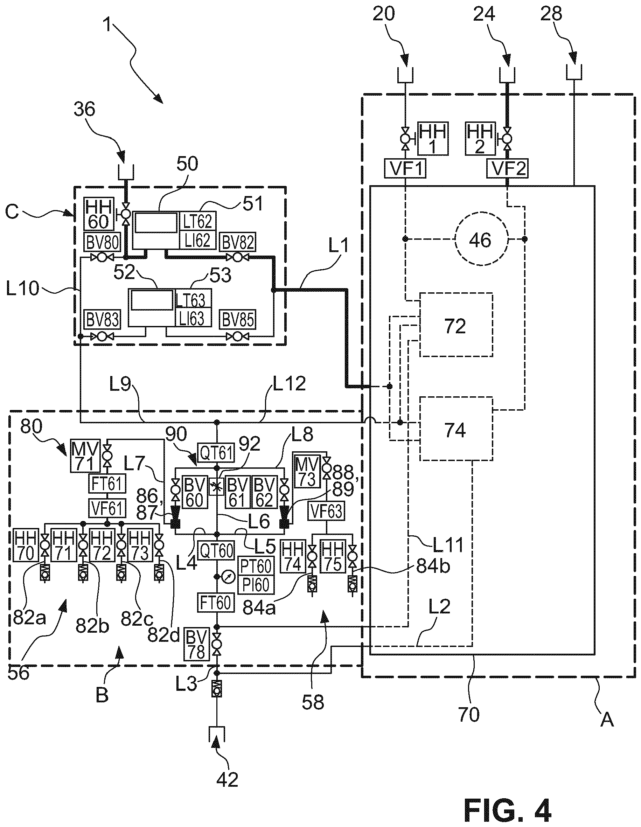

In einem unteren Bereich der Bioreaktorreinigungsanlage 1 sind mehrere Säurekanister 56 und mehrere Basekanister 58 vorgesehen. Die Säurekanister 56 und Basekanister 58 sind jeweils in dafür vorgesehenen Säureschubladen 57 bzw. Baseschubladen 59 untergebracht. Figur 3b zeigt zwei Säurekanister 56 im Detail. An dem Säurekanister 56 ist eine Sauglanze 60 angeordnet, die über weitere Ventile mit dem Säuretank 52 verbindbar ist, wie dies mit Bezug auf Figur 4 noch beschrieben werden wird. Neben der Sauglanze 60, die in einen Sauganschluss 61 eingesetzt wird, ist ein Belüftungsventil 62 vorgesehen. Die Sauglanze 60 hat einen Schraubdeckel 63 (vgl. Figur 3c), einen Lanzenkörper 64 sowie ein Fußventil 65 mit einem fest verbauten Filter. Wenn einer der Kanister 56, 58 gewechselt werden muss, kann die Sauglanze 60 in dafür vorgesehenen Lanzenhalterungen 66a, 66b an den jeweiligen Schubladen eingesteckt werden, sodass diese nicht beschädigt wird.

Figur 4 illustriert nun einen Schaltplan bzw. ein Layout der Bioreaktorreinigungsanlage 1. Der Spannungsanschluss 40 ist dabei ebenso wie die elektronische Steuereinheit 44 nicht gezeigt. Der Schaltplan gemäß Figur 4 ist in drei Systeme aufgeteilt, mit Systemgrenzen A, B und C. Systemgrenze A in der rechten Seite von Figur 4 umfasst neben dem Absauganschluss 20, dem Zuführanschluss 24 sowie dem Hochdruckanschluss 26 eine Pump- und Ventileinheit 70, die hier nur schematisch als Block dargestellt ist, in deren Inneren aber eine Mehrzahl an Ventilen, Rohren, Sensoren und dergleichen angeordnet sein können. Als Teil der Pump- und Ventileinheit 70 ist hier beispielsweise die Pumpe 46 gezeigt, sowie schematisch eine Absaugeinheit 72 und eine Zuführeinheit 74 der Bioreaktorreinigungsanlage 1. Die Absaugeinheit 72 dient zum Absaugen von Flüssigkeit aus dem Bioreaktor 2 und weist dazu den Absauganschluss 20 auf. Die Pumpe 46 wirkt hierauf ein, um ein Vakuum an dem Absauganschluss 20 bereitzustellen. Ferner ist in der Pump- und Ventileinheit 70 die Zuführeinheit 74 ausgebildet, die den Zuführanschluss 24 aufweist.

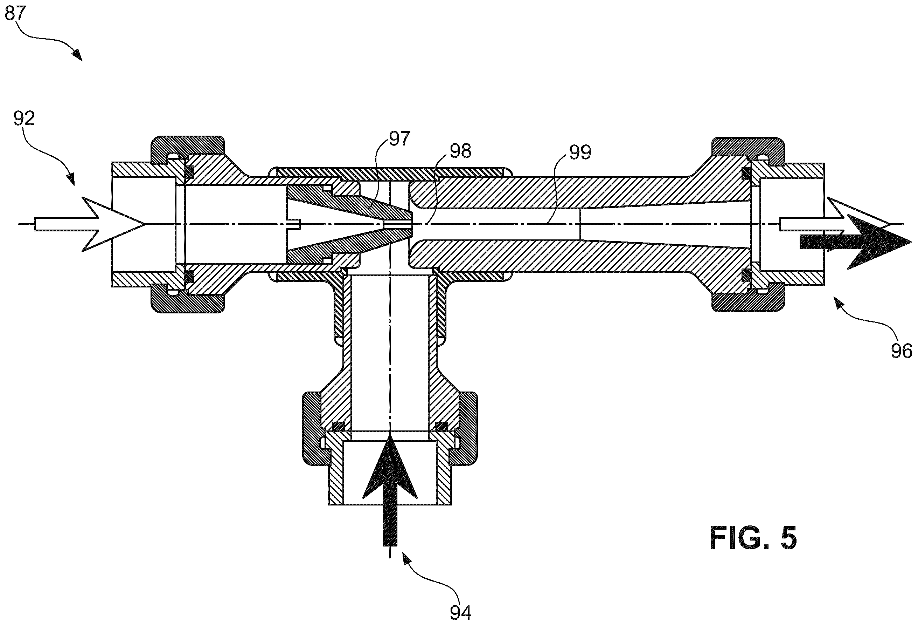

Mit der Systemgrenze B ist eine Dosiereinheit 80 umfasst, sowie die mehreren Säurekanister 56 und mehreren Basekanister 58. Der Frischwasseranschluss 42 ist hier dem System B zugeordnet, genauer gesagt der Dosiereinheit 80. Die Dosiereinheit 80 weist in dem gezeigten Ausführungsbeispiel vier Säurekanister-Anschlüsse 82a, 82b, 82c, 82d sowie zwei Basekanister-Anschlüsse 84a, 84b auf. Die Dosiereinheit 80 ist dann wiederum mit einer Baueinheit in Systemgrenze C verbunden, in der in der hier gezeigten Ausführungsform (Figur 5) der Sammeltank 50 und der Säuretank 52 zugeordnet sind. Ebenso sind der erste Füllstandssensor 51 und der zweite Füllstandssensor 53 dieser Systemgrenze zugeordnet, sowie der Absauganschluss 36. Die Systemgrenzen A, B und C dienen nur zur Illustration, haben aber darüber hinaus keine strukturellen Einflüsse. In bestimmten Ausführungsformen können die Systemgrenzen aber auch bauliche Grenzen bilden.

Im Rahmen einer Reinigung des Bioreaktors 2 kann nun zunächst im ersten Schritt mit der Absaugeinheit 72 in dem Bioreaktor 2, insbesondere dem Flüssigkeitstank 5 vorhandene Restflüssigkeit über den Absauganschluss 20 unter Wirkung der Pumpe 46 abgesaugt werden. Dieses Restfluid wird dann über eine erste Leitung L1 dem Sammeltank 50 zugeführt. Dazu muss ein Ventil BV82, welches vorzugsweise als Kugelventil ausgebildet ist, geöffnet werden, was vorzugsweise durch die elektronische Steuereinheit 44 veranlasst wird. An dem Absauganschluss 20 ist ein erstes Handventil HA1 angeordnet, welches manuell zu öffnen ist. Stromabwärts von diesem in Richtung der Pump- und Ventileinheit 70 ist eine erste Durchflussmesseinheit VF1, die einen kapazitiven Sensor umfasst, angeordnet. Mittels des kapazitiven Sensors der Durchflussmesseinheit VF1 kann die Anwesenheit von Flüssigkeit ermittelt werden, um für den Falk, dass keine Flüssigkeit vorhanden ist, die Leitung zu schließen, oder kein weiteres Vakuum bereitzustellen, bzw. diese an einer anderen Position einzusetzen. Auch diese ist mit der elektronischen Steuereinheit 44 verbunden, um entsprechende Durchflusssignale an dieser bereitzustellen. Wurde nun das Restfluid aus dem Bioreaktor 2 abgesaugt, kann es sinnvoll und notwendig sein, zunächst entweder mittels des Düsenkopfes 10 eine mechanische Reinigung vorzunehmen, oder Flüssigkeit über den 2-Zoll-Schlauch 9 einzubringen, um die Durchlässigkeit des Filterkorbs 8 zu erfassen. Zu diesem Zweck kann beispielsweise über den Zuführanschluss 24 Frischwasser zugeführt werden. Hierzu ist in diesem Ausführungsbeispiel ein zweites Handventil HA2 manuell zu öffnen. Die Zuführeinheit 74 ist über eine zweite Leitung L2 mit dem Frischwasseranschluss 42 verbunden und empfängt von diesem Frischwasser. Die Zuführeinheit 74 kann dann unter Vermittlung der Pumpe 46 diese Flüssigkeit zum Zuführanschluss 24 weiterleiten.

Im Rahmen einer chemischen Reinigung wird der Bioreaktor 2 mittels einer Chemikalie, nämlich insbesondere einerwässrigen Säurelösung, gereinigt. Hierbei kann es erforderlich sein, zunächst die wässrige Säurelösung herzustellen. Dies geschieht in dem hier gezeigten Ausführungsbeispiel mittels der Dosiereinheit 80. Die Dosiereinheit 80 ist über eine dritte Leitung L3 mit dem Frischwasseranschluss 42 verbunden. In der dritten Leitung L3 ist ein Ventil BV78, vorzugsweise Kugelventil, angeordnet, welches mittels der elektronischen Steuereinheit 44 steuerbar ist. Stromabwärts von diesem sind ein erster Durchflusssensor FT60, ein Drucksensor PT60 und ein zweiter pH-Sensor QT60 angeordnet. Stromabwärts von dem zweiten pH-Sensor QT60 verzweigt sich die Leitung in eine vierte Leitung L4, die zu einem Säure-Dosierer 86 führt, eine fünfte Leitung L5, die zu einem Base-Dosierer 88 führt sowie eine sechste Leitung L6, die einen Bypass für Frischwasser zu dem Säure-Dosierer 86 und dem Base-Dosierer 88 bildet. Der Säure-Dosierer 86, der in diesem Ausführungsbeispiel als Säure-Ejektor 87 ausgebildet ist (vgl. Figur 5), empfängt nicht nur Frischwasser über die vierte Leitung L4, sondern ist auch über eine siebte Leitung L7 mit den Säurekanister-Anschlüssen 82a - 82d verbindbar oder verbunden. Genauer gesagt ist die siebte Leitung L7 über ein erstes Magnetventil MV71, einen zweiten Durchflusssensor FT61 und einen dritten Durchflussmesser VF61 mit den Säurekanister-Anschlüssen 82a - 82d verbunden. Der Durchflussmesser VF61 ist wiederum mit einem kapazitiven Sensor ausgestattet und erfasst die Anwesenheit von Flüssigkeit. So kann er als Kanister-leer-Erkennung eingesetzt werden. Der Durchflusssensor FT61 hingegen misst einen Volumenstrom und nutzt vorzugsweise ein Laufrad. Das erste Magnetventil MV71, der zweite Flusssensor FT61 sowie der dritte Durchflussmesser VF61 sind wiederum mit der elektronischen Steuereinheit 44 verbunden, um an dieser Signale bereitzustellen bzw. von dieser gesteuert zu werden. Stromabwärts des Säure-Dosierers 86 ist ein Ventil BV60 angeordnet, welches wiederum vorzugsweise als Kugelventil ausgebildet und von der elektronischen Steuereinheit 44 steuerbar ist.

In analoger Weise ist der Base-Dosierer 88 in diesem Ausführungsbeispiel als Base-Ejektor 89 ausgebildet und wird nicht nur mit Frischwasser über die fünfte Leitung L5 versorgt, sondern auch über eine achte Leitung L8 mit Base. Dazu ist die achte Leitung L8 über ein Ventil MV73, vorzugsweise Kugelventil, und einen Durchflussmesser VF63 mit den ersten und zweiten Basekanister-Anschlüssen 84a, 84b verbunden. Der Durchflussmesser VF63 kann identisch zum Durchflussmesser VF61 ausgebildet. Auch er kann als Kanister-leer-Erkennung eingesetzt werden. Stromabwärts des Base-Dosierers 88 ist wiederum ein Ventil BV62 angeordnet, welches vorzugsweise als Kugelventil ausgebildet und von der elektronischen Steuereinheit 44 steuerbar ist.

Der Säure-Ejektor 87, der Base-Ejektor 89 und die sechste Leitung L6 münden gemeinsam in einen Mischer 90. Zwischen der sechsten Leitung L6 und dem Mischer 90 ist gemäß dem hier gezeigten Ausführungsbeispiel eine optionale Drossel 92 angeordnet, die über ein Stellglied BV61 einstellbar ist. Das Stellglied BV61 ist ebenfalls von der elektronischen Steuereinheit 44 steuerbar. Auf diese Weise lassen sich durch Steuerung der Ventile BV60, BV62 bzw. der Drossel 92 über das Stellglied BV61 die Durchflussmengen von mit Säure versetztem Frischwasser über die vierte Leitung L4, Frischwasser über die sechste Leitung L6 und mit Base versetztem Frischwasser über die fünfte Leitung L5 bedarfsgerecht zusammenmischen. Stromabwärts des Mischers 90 ist ein erster pH-Sensor QT61 angeordnet, welcher ein erstes pH-Signal an der elektronischen Steuereinheit 44 bereitstellt. Der Mischer 90 ist über eine neunte Leitung L9 und ein weiteres vorzugsweise als Kugelventil ausgebildetes Ventil BV83 mit dem Säuretank 52 verbunden. Über den ersten pH-Sensor QT61 kann also der pH-Wert der Flüssigkeit erfasst werden, die dem Säuretank 52 über das Ventil BV83 zugeführt wird. Alternativ kann ausgehend von dem Mischer 90 die Flüssigkeit auch dem Sammeltank 50 zugeführt werden. Zu diesem Zweck ist ein Ventil BV80 vorgesehen, welches eine zehnte Leitung L10, die von der neunten Leitung L9 abzweigt, mit dem Sammeltank 50 verbindet.

Auf diese Weise lässt sich eine wässrige Säurelösung in dem Säuretank 52 erzeugen, die dann zum Reinigen des Bioreaktors 2 verwendet werden kann. Um die wässrige Säurelösung aus dem Säuretank 52 dem Bioreaktor 2 zuzuführen, wird das Ventil BV83 geschlossen und das Ventil BV85, welches zwischen den Säuretank 52 und die erste Leitung L1 gesetzt ist, geöffnet. Anschließend kann unter Vermittlung der Pumpe 46 ein Unterdruck in der ersten Leitung L1 aufgebaut werden und die wässrige Säurelösung über das Ventil BV85, die erste Leitung L1 und die Zuführeinheit 74 dem Bioreaktor 2 über den Zuführanschluss 24 zugeführt werden.

Die wässrige Säurelösung wird dann durch den Bioreaktor 2 fließen, genauer gesagt durch den Filterkorb 8 und anschließend in den Flüssigkeitstank 5 hinein. Auf diesem Weg reagiert die Säure der wässrigen Säurelösung mit anhaftenden Kalkablagerungen und löst diese. Es ist auch möglich, hierbei gleichzeitig Luftblasen einzuperlen, um die Reinigungswirkung zu erhöhen. Dies ist insbesondere für Leitungen des Bioreaktors 2 bevorzugt, die ebenfalls gereinigt werden können und sollen.

Nachdem nun die Flüssigkeit durch den Bioreaktor 2 gelaufen ist, kann diese über den 1-Zoll-Anschluss 11 abgesaugt werden. Dies erfolgt dann über die Absaugeinheit 74, vermittels der Pumpe 46.

Die so abgesaugte Flüssigkeit aus dem Bioreaktor 2 kann dann von der Absaugeinheit 72 entweder direkt über die erste Leitung L1 und das Ventil BV85 wiederum dem Säuretank 52 zugeführt werden. Es ist allerdings auch möglich, die abgesaugte Flüssigkeit ausgehend von der Absaugeinheit 72 über eine elfte Leitung L11 in die dritte Leitung L3 einzuspeisen, und zwar in dem hier gezeigten Ausführungsbeispiel (Figur 4) zwischen dem Ventil BV78 und dem Durchflusssensor FT60. Bleiben die Ventile BV60, BV62 geschlossen, kann die abgesaugte Flüssigkeit über den zweiten pH-Sensor QT60, die Drossel 92 und den ersten pH-Sensor 61 geleitet werden, dann über die neunte Leitung L9, das Ventil BV83 und in den Säuretank 52. In anderen Ausführungsformen kann auch vorgesehen sein, dass die Leitung L11 an anderen Stellen in die dritte, die sechste oder die neunte Leitung L3, L6, L9 mündet, beispielsweise könnte die elfte Leitung L11 auch stromabwärts des zweiten pH-Sensors QT60 in die sechste Leitung L6 münden, beispielsweise zwischen der Drossel 92 und dem ersten pH-Sensor 90. Es könnte auch vorgesehen sein, dass in der ersten Leitung L1 ein weiterer pH-Wert vorgesehen ist, um den pH-Wert der aus dem Bioreaktor 2 abgesaugten Flüssigkeit unmittelbar zu messen.

Solange der pH-Wert der abgesaugten Flüssigkeit, die an dem ersten pH-Sensor QT61 vorbeigeführt wird, einen vorbestimmten pH-Schwellwert unterschreitet, kann die wässrige Säurelösung nochmals zum Reinigen des Bioreaktors 2 oder eines weiteren Bioreaktors verwendet werden. Der pH-Wert der abgesaugten Flüssigkeit wird hier als Säurequalitätswert verwendet. Andere Werte können ebenso verwendet werden, wie insbesondere eine Beladung der Flüssigkeit mit Schmutzpartikeln oder dergleichen.

Wird festgestellt, dass der erste pH-Wert, der von dem ersten pH-Sensor QT61 erfasst wird, einen vorbestimmten pH-Schwellwert überschreitet, sollte die wässrige Säurelösung in dem Säuretank 52 neutralisiert werden, um anschließend entsorgt werden zu können. Um eine Neutralisierung durchzuführen, wird die abgesaugte Flüssigkeit entweder von dem Absauganschluss 20 direkt, oder aus dem Säuretank 52 heraus mittels der Absaugeinheit 72 über die elfte Leitung L11 der Dosiereinheit 80 zugeführt. Hierbei wird die zu neutralisierende wässrige Säurelösung an dem Flusssensor FT60 vorbeigeführt, dem zweiten pH-Sensor QT60 und dann zwischen den vierten, fünften und sechsten Leitungen L4, L5, L6 aufgeteilt. Hierbei wird über den Base-Dosierer 88 Base zudosiert, sodass der pH-Wert der zu neutralisierenden wässrigen Säurelösung erhöht wird. Der erhöhte pH-Wert kann seinerseits mit dem ersten pH-Sensor QT61 gemessen werden. Die Flüssigkeit kann dann wiederum in den Säuretank 52 oder über eine zwölfte Leitung L12 zurück zur Absaugeinheit 72 und von dort aus wieder der elften Leitung L11 zugeführt werden. Die wässrige Säurelösung wird also neutralisiert, während sie innerhalb der Bioreaktorreinigungsanlage 1 zirkuliert und auf diese Weise wird eine Durchflussneutralisierung durchgeführt. Sobald ein ausreichend hoher pH-Wert erreicht wurde, wird die Flüssigkeit nicht über das Ventil BV83 zurück in den Säuretank 82 geführt, sondern über das Ventil BV80 in den Sammeltank 50, um von dort entsorgt werden zu können.

Neben dem Säure-Ejektor 87 und dem Base-Ejektor 89 könnten auch ein oder mehrere weitere Ejektoren vorgesehen sein, um beispielsweise Blauwassergemische für chemische Toiletten in Zügen mittels der Bioreaktorreinigungsanlage 1 herzustellen.

Figur 5 illustriert einen Ejektor, wie er als Säure-Ejektor 87 und Base-Ejektor 89 eingesetzt wird. Beispielhaft illustriert Figur 5 dies anhand des Säure-Ejektors 87. Gleiches gilt aber auch für den Base-Ejektor 89. Der Säure-Ejektor 87 hat einen ersten Einlass 92, der mit der vierten Leitung L4 verbunden ist und Frischwasser unter Druck empfängt. Er weist ferner einen zweiten Einlass 94 auf, der mit der siebten Leitung L7 verbunden ist und somit Säure empfängt. Der Ejektor hat dann einen Ausgang 96, der mit dem Ventil BV60 verbunden ist (vgl. Figur 4). Im Inneren ist ein Düsenstück 97 vorgesehen, das nach Art einer Venturi-Pumpe in einen Zwischenraum 98 mündet, um so Säure von dem zweiten Eingang 94 anzusaugen und in eine gemeinsame Ejektorkammer 99 zu befördern. Diese führt dann zum Ausgang 96, an dem Frischwasser und Säure gemischt vorliegen. Zur genauen Dosierung der Säure am zweiten Einlass 94 kann das Ventil MV71 gepulst geöffnet werden, um den pH-Wert der gemischten Flüssigkeit am Ausgang 96 möglichst genau einzustellen.

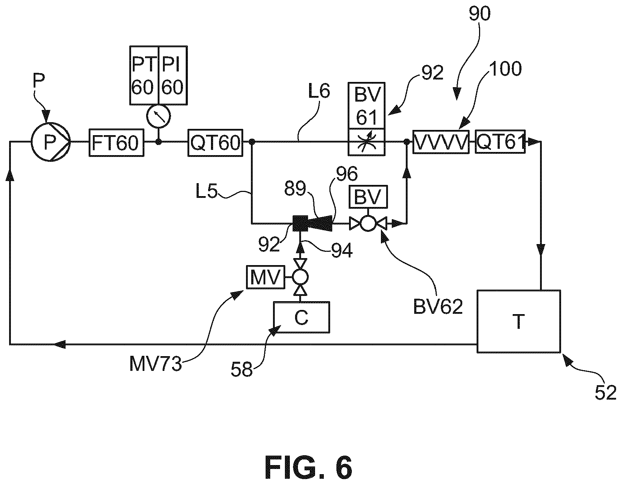

Figur 6 illustriert den Kreislauf der Durchlaufneutralisierung schematisch. Mit P ist dort entweder die Pumpe 46 oder der Frischwasseranschluss 42 bezeichnet. Wichtig ist hier nur, dass der Kreislauf in Gang gesetzt wird. Von dort aus gelangt die Flüssigkeit dann zum ersten Durchflussmesser FT60, zum Drucksensor PT60 sowie zum zweiten pH-Sensor QT60. Die Leitung verzweigt sich dann einerseits in die Leitung L6 und die Leitung L5, die zum Base-Ejektor 89 führt, genauer gesagt zum ersten Einlass 92. An den zweiten Einlass 94 ist hier das Ventil MV73 angeschlossen, über welches in dem in Figur 6 gezeigten Ausführungsbeispiel dann Base dem zweiten Einlass 94 zugeführt werden kann. Über das Ventil BV62 kann dann die Dosierung weiter eingestellt werden, während in der sechsten Leitung L6 über die Drossel 92 und das Ventil BV61 der andere Pfad reguliert werden kann.

In dem Mischer 90, in dem die sechste Leitung L6 und die fünfte Leitung L5 sowie auch die vierte Leitung L4 (vgl. Figur 4) zusammengeführt werden, ist in dem hier gezeigten Ausführungsbeispiel zusätzlich ein Diffusor 100 vorgesehen. Dieser ist hier nur schematisch dargestellt und kann beispielsweise als statischer Mischer mit verschiedenen Mischelementen ausgebildet sein. Stromabwärts von dem Diffusor 100 ist hier noch der erste pH-Sensor QT61 vorgesehen. Dieser Kreislauf wird so lange ausgeführt, bis in dem Säuretank 52 ein ausreichend hoher pH-Wert erreicht wurde, um die wässrige Lösung entsorgen zu können.

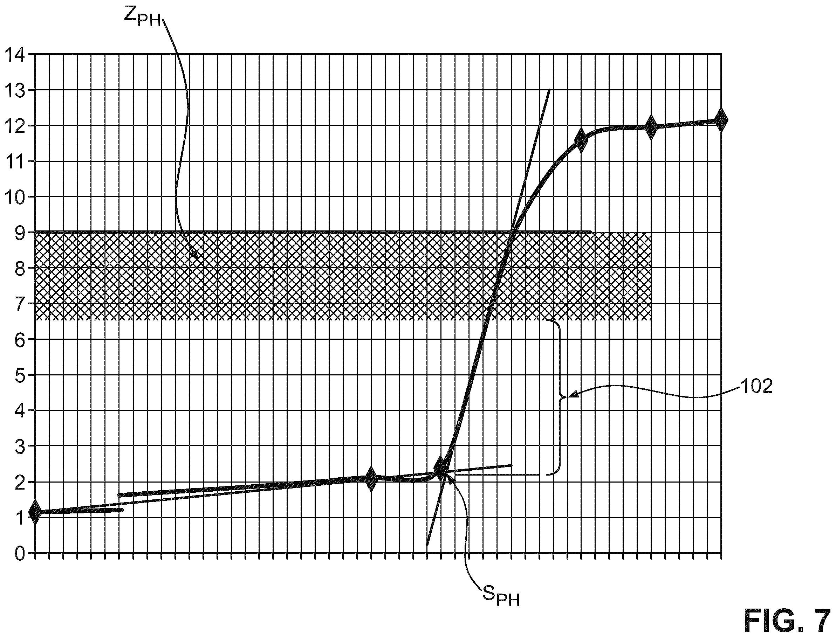

Figur 7 illustriert nun eine charakteristische Kurve einer Neutralisierung einer wässrigen Säurelösung. Auf der Ordinate ist der pH-Wert eingetragen, an der Abszisse die zugegebene Menge an Base. Wie sich aus dieser Grafik leicht entnehmen lässt, steigt der pH-Wert zunächst auch bei substanziell zugegebener Menge in einem ersten Abschnitt bis etwa pH 2 nur leicht an, dann sehr stark, bis etwas pH 11/12, um dann wieder flach zu verlaufen. Der neutrale Bereich zwischen 6,5 und 9 ist hier hinterlegt angezeichnet und der Ziel-pH-Wert ZpH liegt ebenfalls hier. In diesem Bereich sollte die wässrige Lösung sein, um entsorgt werden zu können. Diese Grafik verdeutlicht, warum es schwierig ist, in einem stationären Prozess innerhalb des Säuretanks 52 eine Neutralisierung herbeizuführen. Bei der Durchlaufneutralisierung, wie sie im Rahmen der vorliegenden Erfindung vorgeschlagen wird, kann der neutrale Bereich zwischen 6,5 und 9 pH durch den geschlossenen Regelkreis nach und nach eingestellt werden, ohne ein "Überschwingen" zu provozieren. In dem Diagramm ist auch der pH-Schwellwert SpH eingezeichnet, anhand dessen gemessen wird, ob die wässrige Säurelösung in dem Säuretank 52 weiterhin zur Reinigung verwendet werden kann oder nicht. Wird festgestellt, dass die wässrige Lösung im Säuretank 52 den pH-Schwellwert SpH überschreitet, wird der Neutralisationsprozess eingeleitet. In einem Bereich 102 ist die Lösung zwar nicht mehr für die Reinigung verwendbar, aber auch noch nicht neutral, um entsorgt werden zu können.

## Patentansprüche

1. Bioreaktorreinigungsanlage (1) zum Reinigen eines Bioreaktors (2), vorzugsweise eines Bioreaktors in einem Schienenfahrzeug, mit
einer Absaugeinheit (72) zum Absaugen von Flüssigkeit aus dem Bioreaktor (2), wobei die Absaugeinheit (72) einen Absauganschluss (20) zum Anschließen an den Bioreaktor (2) aufweist;
einer Zuführeinheit (74) zum Zuführen von Flüssigkeit in den Bioreaktor (2), wobei die Zuführeinheit (74) wenigstens einen Zuführanschluss (24) zum Anschließen an den Bioreaktor (2) aufweist;
einer elektronischen Steuereinheit (44) zum Ansteuern der Absaugeinheit (72) und der Zuführeinheit;
einem Säuretank (52) zum Aufnehmen einer wässrigen Säurelösung;
einem Sammeltank (50) zum Aufnehmen von aus dem Bioreaktor abgesaugter Flüssigkeit; und
einem Frischwasseranschluss (42) zum Versorgen der Bioreaktorreinigungsanlage (1) mit Frischwasser;
wobei die Zuführeinheit (74) mit dem Frischwasseranschluss (42) und dem Säuretank (52) verbunden ist, um wahlweise Frischwasser und/oder wässrige Säurelösung zu dem Zuführanschluss (24) bereitzustellen,
und wobei die Absaugeinheit (72) zum Absaugen von Flüssigkeit aus dem Bioreaktor (2) wahlweise mit dem Säuretank (52) oder dem Sammeltank (50) verbindbar ist, um aus dem Bioreaktor (2) abgesaugte Säurelösung dem Säuretank (52) und aus dem Bioreaktor (2) abgesaugtes im Wesentlichen pH-neutrales Restfluid dem Sammeltank (50) zuzuführen;
und mit einer Dosiereinheit (80), die
eine oder mehrere Säurekanister-Anschlüsse (82a, 82b, 82c, 82d) zum Anschließen von Säurekanistern (56) und einen oder mehrere Basekanister-Anschlüsse (84a, 84b) zum Anschließen von Basekanistern (58) aufweist,
einen Säure-Dosierer (86), der eingangsseitig mit dem einen oder den mehreren Säurekanister-Anschlüssen (82a, 82b, 82c, 82d) und ausgangsseitig mit dem Säuretank (52) verbindbar ist, und einen Base-Dosierer (88) aufweist, der eingangsseitig mit dem einen oder den mehreren Basekanister-Anschlüssen (84a, 84b) und ausgangsseitig mit dem Säuretank (52) verbindbar ist, sowie
einen Mischer aufweist, wobei der Mischer eingangsseitig mit einem Frischwasseranschluss und dem Säure-Dosierer (86) und/oder Base-Dosierer (88) und ausgangsseitig mit dem Säuretank verbindbar ist, um diesem eine zum Neutralisieren der in dem Säuretank (52) vorhandenen Säurelösung nutzbare Lösung zuzuführen.

2. Bioreaktorreinigungsanlage nach Anspruch 1, aufweisend einen ersten pH-Sensor (QT61) zum Erfassen eines ersten pH-Werts einer dem Säuretank (52) zugeführten Flüssigkeit und zum Bereitstellen eines ersten pH-Signals an der elektronischen Steuereinheit (44), und/oder aufweisend einen zweiten pH-Sensor (QT60) zum Erfassen eines zweiten pH-Werts einer über den Absauganschluss (20) abgesaugten Flüssigkeit.

3. Bioreaktorreinigungsanlage nach einem der vorstehenden Ansprüche, wobei der Säure-Dosierer (86) einen Säure-Ejektor (87) aufweist, der eingangsseitig mit dem Frischwasseranschluss (42) verbindbar ist, und wobei der Base-Dosierer (88) vorzugsweise einen Base-Ejektor (89) aufweist, der eingangsseitig mit dem Frischwasseranschluss (42) verbindbar ist.

4. Bioreaktorreinigungsanlage nach einem der vorstehenden Ansprüche, wobei der Mischer (90) eine Drossel (92) aufweist, zum Drosseln des dem Mischer (90) zugeführten Frischwassers.

5. Bioreaktorreinigungsanlage nach einem der vorstehenden Ansprüche, aufweisend einen ersten Durchflussmesser (FT60) zum Messen des dem Mischer (90) und/oder dem Säure-Dosierer (86) und/oder dem Base-Dosierer (88) zugeführten Frischwasser, und vorzugsweise aufweisend einen zweiten Durchflussmesser (FT61) zum Messen der dem Säure-Dosierer (86) zugeführten Säure.

6. Bioreaktorreinigungsanlage nach einem der vorstehenden Ansprüche, wobei die Absaugeinheit (72) und/oder die Zuführeinheit (74) betreibbar sind zum Pumpen von Flüssigkeit aus dem Säuretank (52) und/oder Bioreaktor (2) heraus zu dem Mischer (90) und zurück in den Säuretank (52) und/oder Bioreaktor (2).

7. Bioreaktorreinigungsanlage nach Anspruch 2, wobei die Absaugeinheit (72) und/oder die Zuführeinheit (74) betreibbar sind zum Pumpen von Flüssigkeit aus dem Säuretank (52) und/oder Bioreaktor (2) heraus zu dem ersten und/oder zweiten pH-Sensor (QT60, QT61) und zurück in den Säuretank (52) und/oder Bioreaktor (2).

8. Bioreaktorreinigungsanlage nach einem der vorstehenden Ansprüche, wobei die elektronische Steuereinheit (44) ausgebildet ist zum:
- Ermitteln eines eine Qualität von in dem Säuretank (52) vorhandener wässriger Säurelösung angebenden Säurequalitätswerts;
- Vergleichen des ermittelten Säurequalitätswerts mit einem vorbestimmten Säurevergleichswert; und
- in Abhängigkeit des Vergleichens: Einleiten einer Neutralisation der wässrigen Säurelösung in dem Säuretank (52).

9. Bioreaktorreinigungsanlage nach Anspruch 8, wobei der Säurequalitätswert der erste pH-Wert ist, und die elektronische Steuereinheit (44) ausgebildet ist zum:
- Ermitteln des ersten pH-Werts von in dem Säuretank (52) vorhandener wässriger Säurelösung;
- Vergleichen des ermittelten ersten pH-Werts mit einem vorbestimmten pH-Schwellwert (SpH); und für den Fall, dass der ermittelte erste pH-Wert den pH-Schwellwert (SpH) überschreitet:
- Einleiten einer Neutralisation der wässrigen Säurelösung in dem Säuretank (52).

10. Bioreaktorreinigungsanlage nach einem der Ansprüche 8 bis 9, wobei die elektronische Steuereinheit (44) dazu ausgebildet ist, zum Neutralisieren der in dem Säuretank (52) vorhandenen wässrigen Säurelösung, während die Absaugeinheit (72) und/oder die Zuführeinheit (74) die Flüssigkeit aus dem Säuretank (52) heraus zu dem ersten oder zweiten pH-Sensor (QT60, QT61) und zurück in den Säuretank (52) pumpen, den Base-Dosierer (88) zu veranlassen Base in den Mischer (90) auszugeben, bis in dem Säuretank (52) ein neutraler Ziel-pH-Wert (ZpH) erreicht ist.

11. Verfahren zum Betreiben einer Bioreaktorreinigungsanlage (1), vorzugsweise einer Bioreaktorreinigungsanlage (1) nach einem der vorstehenden Ansprüche, umfassend die Schritte:
- Ermitteln eines eine Qualität von in einem Säuretank (52) der Bioreaktorreinigungsanlage (1) vorhandener wässriger Säurelösung angebenden Säurequalitätswerts;
- Vergleichen des ermittelten Säurequalitätswerts mit einem vorbestimmten Säurevergleichswert; und
- in Abhängigkeit von dem Vergleichen:
- Einleiten einer Neutralisation der wässrigen Säurelösung in dem Säuretank (52).

12. Verfahren nach Anspruch 11, wobei der Säurequalitätswert ein erster pH-Wert ist, und wobei das Verfahren die Schritte umfasst:
- Ermitteln des ersten pH-Werts von in dem Säuretank (52) vorhandener wässriger Säurelösung;
- Vergleichen des ermittelten ersten pH-Werts mit einem vorbestimmten pH-Schwellwert (SpH); und für den Fall, dass der ermittelte erste pH-Wert den pH-Schwellwert (SpH) überschreitet:
- Einleiten der Neutralisation der wässrigen Säurelösung in dem Säuretank (52).

13. Verfahren nach einem der Ansprüche 11 bis 12, umfassend:
- Pumpen von Flüssigkeit aus dem Säuretank (52) heraus zu einem Mischer (90) der Bioreaktorreinigungsanlage (1) und zurück in den Säuretank (52);und
- Einmischen in dem Mischer (90) von Säure und/oder Base und/oder Frischwasser; und/oder
- Ermitteln eines ersten pH-Werts stromabwärts des Mischers (90); und/oder
- Ermitteln eines zweiten pH-Werts stromaufwärts des Mischers (90); und
- in Abhängigkeit des ersten und/oder zweiten pH-Werts, Einmischen in dem Mischer (90) von Säure und/oder Base zum Erzielen eines neutralen Ziel-pH-Werts (ZpH) in dem Säuretank (52); und
- wenn der neutrale Ziel-pH-Wert erreicht ist: Pumpen der Flüssigkeit aus dem Säuretank (52) in einen Sammeltank (50) der Bioreaktorreinigungsanlage (1) und/oder einen Entsorgungskanal.

14. Verfahren nach einem der Ansprüche 11 bis 13, umfassend:
- in Abhängigkeit des Vergleichens: Zuführen von Flüssigkeit aus dem Säuretank (52) zu dem Bioreaktor (2) zum Reinigen des Bioreaktors (2); und
- Absaugen von Flüssigkeit aus dem Bioreaktor (2) und Zuführen der abgesaugten Flüssigkeit zu dem Säuretank (52).

15. Computerprogrammprodukt, umfassend Codemittel, die wenn auf einem Computer ausgeführt, eine Bioreaktorreinigungsanlage (1) veranlassen, die Schritte gemäß dem Verfahren nach einem der Ansprüche 11 bis 14 auszuführen.

## Claims

1. A bioreactor cleaning system (1) for cleaning a bioreactor (2), preferably a bioreactor in a rail vehicle, with
a suction unit (72) for suctioning liquid from the bioreactor (2), the suction unit (72) having a suction connection (20) for connecting to the bioreactor (2);
a supply unit (74) for supplying liquid into the bioreactor (2), the supply unit (74) having at least one supply connection (24) for connecting to the bioreactor (2);
an electronic control unit (44) for controlling the suction unit (72) and the supply unit;
an acid tank (52) for receiving an aqueous acid solution;
a collection tank (50) for receiving liquid suctioned from the bioreactor; and
a fresh water connection (42) for supplying the bioreactor cleaning system (1) with fresh water;
wherein the supply unit (74) is connected to the fresh water port (42) and the acid tank (52) to selectively provide fresh water and/or aqueous acid solution to the supply connection (24),
and wherein the suction unit (72) for suctioning liquid from the bioreactor (2) is selectively connectable to the acid tank (52) or the collection tank (50) for supplying acid solution suctioned from the bioreactor (2) to the acid tank (52) and for supplying substantially pH-neutral residual fluid suctioned from the bioreactor (2) to the collection tank (50);
and with a dosing unit (80), which
has one or more acid canister ports (82a, 82b, 82c, 82d) for connecting acid canisters (56) and one or more base canister ports (84a, 84b) for connecting base canisters (58),
an acid doser (86) connectable on the input side to the one or more acid canister ports (82a, 82b, 82c, 82d) and on the output side to the acid tank (52), and a base doser (88) connectable on the input side to the one or more base canister ports (84a, 84b) and on the output side to the acid tank (52), and
a mixer, wherein the mixer can be connected on the input side to a fresh water connection and the acid dosing unit (86) and/or base dosing unit (88), and on the output side to the acid tank in order to supply the acid tank with a solution usable to neutralize the acid solution present in the acid tank (52).

2. The bioreactor cleaning system of claim 1, comprising a first pH sensor (QT61) for detecting a first pH value of a fluid supplied to the acid tank (52) and for providing a first pH signal to the electronic control unit (44) and/or comprising a second pH sensor (QT60) for detecting a second pH value of a liquid suctioned via the suction connection (20).

3. The bioreactor cleaning system according to any one of the preceding claims, wherein the acid doser (86) comprises an acid ejector (87) which can be connected to the fresh water connection (42) on the inlet side, and wherein the base doser (88) comprises preferably a base ejector (89) which can be connected to the fresh water connection (42) on the inlet side.

4. The bioreactor cleaning system according to any one of the preceding claims, wherein the mixer (90) comprises a throttle (92) for throttling the fresh water supplied to the mixer (90).

5. The bioreactor cleaning system according to any one of the preceding claims, comprising a first flow meter (FT60) for measuring the fresh water supplied to the mixer (90) and/or the acid doser (86) and/or the base doser (88), and preferably comprising a second flow meter (FT61) for measuring the acid supplied to the acid doser (86).

6. The bioreactor cleaning system according to any one of the preceding claims, wherein the suction unit (72) and/or the supply unit (74) are operable to pump liquid out of the acid tank (52) and/or bioreactor (2) to the mixer (90) and back into the acid tank (52) and/or bioreactor (2).

7. The bioreactor cleaning system according to claim 2, wherein the suction unit (72) and/or the supply unit (74) are operable to pump liquid out of the acid tank (52) and/or bioreactor (2) to the first and/or second pH sensor (QT60, QT61) and back into the acid tank (52) and/or bioreactor (2).

8. The bioreactor cleaning system according to any one of the preceding claims, wherein the electronic control unit (44) is adapted to:
- determine an acid quality value indicating a quality of aqueous acid solution present in the acid tank (52);
- compare the determined acid quality value to a predetermined acid comparison value; and
- depending on the comparison: initiate neutralization of the aqueous acid solution in the acid tank (52).

9. The bioreactor cleaning system according to claim 8, wherein the acid quality value is the first pH value, and the electronic control unit (44) is adapted to:
- determine the first pH value of aqueous acid solution present in the acid tank (52);
- compare the determined first pH value with a predetermined pH threshold value (SpH); and in the event that the determined first pH value exceeds the pH threshold value (SpH):
- initiate neutralization of the aqueous acid solution in the acid tank (52).

10. The bioreactor cleaning system according to any one of claims 8 to 9, wherein the electronic control unit (44) is adapted to neutralize the aqueous acid solution present in the acid tank (52), while the suction unit (72) and/or the supply unit (74) pump the liquid out of the acid tank (52) to the first or second pH sensor (QT60, QT61) and back into the acid tank (52), to cause the base doser (88) to dispense base into the mixer (90) until a neutral target pH (ZpH) is reached in the acid tank (52).

11. A method for operating a bioreactor cleaning system (1), preferably a bioreactor cleaning system (1) according to any of the preceding claims, comprising the steps:
- determining an acid quality value indicating a quality of an aqueous acid solution present in an acid tank (52) of the bioreactor cleaning system (1);
- comparing the determined acid quality value to a predetermined acid comparison value; and
- depending on the comparison:
- initiate neutralization of the aqueous acid solution in the acid tank (52).

12. The method of claim 11, wherein the acid quality value is a first pH-value, and wherein the method comprises the steps of:
- determining the first pH-value of aqueous acid solution present in the acid tank (52);
- comparing the determined first pH-value with a predetermined pH threshold value (SpH); and in the event that the determined first pH-value exceeds the pH threshold value (SpH):
- initiate neutralization of the aqueous acid solution in the acid tank (52).

13. The method according to any one of claims 11 to 12, comprising:
- pumping liquid out of the acid tank (52) to a mixer (90) of the bioreactor cleaning system (1) and back into the acid tank (52); and
- mixing in the mixer (90) acid and/or base and/or fresh water; and/or
- determining a first pH-value downstream of the mixer (90); and/or
- determining a second pH-value upstream of the mixer (90); and
- depending on the first and/or second pH-value, mixing in the mixer (90) acid and/or base to achieve a neutral target pH (ZpH) in the acid tank (52); and
- when the neutral target pH is reached: pumping the liquid from the acid tank (52) into a collection tank (50) of the bioreactor cleaning system (1) and/or a disposal drain.

14. The method according to any one of claims 11 to 13, comprising:
- dependent on the comparison: supplying liquid from the acid tank (52) to the bioreactor (2) for cleaning the bioreactor (2); and
- extracting liquid from the bioreactor (2) and supplying the extracted liquid to the acid tank (52).

15. A computer program product comprising code means which, when executed on a computer, causes a bioreactor cleaning system (1) to perform the steps according to the method of any one of claims 11 to 14.

## Revendications

1. Installation de nettoyage (1) de bioréacteur destinée à nettoyer un bioréacteur (2), de préférence un bioréacteur dans un véhicule ferroviaire, comprenant une unité d'aspiration (72) destinée à aspirer du liquide depuis le bioréacteur (2), l'unité d'aspiration (72) présentant un raccord d'aspiration (20) pour le raccordement au bioréacteur (2);
une unité d'alimentation (74) destinée à alimenter du liquide dans le bioréacteur (2), l'unité d'alimentation (74) comprenant au moins un raccord d'alimentation (24) pour le raccordement au bioréacteur (2);
une unité de commande électronique (44) destinée à commander l'unité d'aspiration (72) et l'unité d'alimentation;
un réservoir d'acide (52) destiné à recevoir une solution acide aqueuse;
un réservoir de collecte (50) destiné à recevoir du liquide aspiré à partir du bioréacteur; et
un raccord d'eau fraîche (42) destiné à alimenter l'installation de nettoyage (1) de bioréacteur en eau fraîche;
dans laquelle l'unité d'alimentation (74) est connectée au raccord d'eau fraîche (42) et au réservoir d'acide (52) afin de fournir au choix de l'eau fraîche et/ou une solution acide aqueuse au raccord d'alimentation (24),
et dans laquelle l'unité d'aspiration (72) destinée à aspirer du liquide depuis le bioréacteur (2) peut être reliée au choix au réservoir d'acide (52) ou au réservoir de collecte (50), afin d'amener la solution acide aspirée du bioréacteur (2) au réservoir d'acide (52) et du fluide résiduel, au pH sensiblement neutre, aspiré du bioréacteur (2) au réservoir de collecte (50);
et comprenant une unité de dosage (80) qui
comporte un ou plusieurs raccords de bidons d'acide (82a, 82b, 82c, 82d) pour raccorder des bidons d'acide (56) et un ou plusieurs raccords de bidons de base (84a, 84b) pour raccorder des bidons de base (58),
un doseur d'acide (86), qui peut être relié côté entrée au un ou aux plusieurs raccords de bidons d'acide (82a, 82b, 82c, 82d) et côté sortie au réservoir d'acide (52), et un doseur de base (88), qui peut être relié côté entrée au un ou aux plusieurs raccords de bidons de base (84a, 84b) et côté sortie au réservoir d'acide (52), et
un mélangeur, le mélangeur pouvant être relié, côté entrée, à un raccord d'eau fraîche et au doseur d'acide (86) et/ou au doseur de base (88) et, côté sortie, au réservoir d'acide, afin d'amener à celui-ci une solution utilisable pour neutraliser la solution acide présente dans le réservoir d'acide (52).

2. Installation de nettoyage de bioréacteur selon la revendication 1, comprenant un premier capteur de pH (QT61) destiné à détecter une première valeur de pH d'un liquide amené au réservoir d'acide (52) et à fournir un premier signal de pH à l'unité de commande électronique (44), et/ou comprenant un deuxième capteur de pH (QT60) destiné à détecter une deuxième valeur de pH d'un liquide aspiré via le raccord d'aspiration (20).

3. Installation de nettoyage de bioréacteur selon l'une quelconque des revendications précédentes, dans laquelle le doseur d'acide (86) comporte un éjecteur d'acide (87) qui peut être relié côté entrée au raccord d'eau fraîche (42), et dans laquelle le doseur de base (88) comporte de préférence un éjecteur de base (89) qui peut être relié coté entrée au raccord d'eau fraîche (42).

4. Installation de nettoyage de bioréacteur selon l'une quelconque des revendications précédentes, dans laquelle le mélangeur (90) comprend un papillon d'obturation (92) pour obturer l'eau fraîche amenée au mélangeur (90).

5. Installation de nettoyage de bioréacteur selon l'une quelconque des revendications précédentes, comprenant un premier débitmètre (FT60) destiné à mesurer l'eau fraîche amenée au mélangeur (90) et/ou au doseur d'acide (86) et/ou au doseur de base (88), et comprenant de préférence un deuxième débitmètre (FT61) destiné à mesurer l'acide amené au doseur d'acide (86).

6. Installation de nettoyage de bioréacteur selon l'une quelconque des revendications précédentes, dans laquelle l'unité d'aspiration (72) et/ou l'unité d'alimentation (74) peuvent/peut être utilisée(s) pour pomper du liquide depuis le réservoir d'acide (52) et/ou le bioréacteur (2) vers le mélangeur (90) et en retour dans le réservoir d'acide (52) et/ou le bioréacteur (2).

7. Installation de nettoyage de bioréacteur selon la revendication 2, dans laquelle l'unité d'aspiration (72) et/ou l'unité d'alimentation (74) peuvent/peut être utilisée(s) pour pomper du liquide depuis le réservoir d'acide (52) et/ou le bioréacteur (2) vers le premier et/ou le deuxième capteur de pH (QT60, QT61) et en retour dans le réservoir d'acide (52) et/ou le bioréacteur (2).

8. Installation de nettoyage de bioréacteur selon l'une quelconque des revendications précédentes, dans laquelle l'unité de commande électronique (44) est conçue pour:
- déterminer une valeur de qualité d'acide indiquant une qualité de solution acide aqueuse présente dans le réservoir d'acide (52);
- comparer la valeur de qualité d'acide déterminée à une valeur de comparaison d'acide prédéterminée; et,
- en fonction de la comparaison: déclencher une neutralisation de la solution acide aqueuse dans le réservoir d'acide (52).

9. Installation de nettoyage de bioréacteur selon la revendication 8, dans laquelle la valeur de qualité d'acide est la première valeur de pH, et l'unité de commande électronique (44) est conçue pour:
- déterminer la première valeur de pH de la solution acide aqueuse présente dans le réservoir d'acide (52);
- comparer la première valeur de pH déterminée à une valeur de pH seuil (SpH) prédéterminée; et dans le cas où la première valeur de pH déterminée est supérieure à la valeur de pH seuil (SpH):
- déclencher une neutralisation de la solution acide aqueuse dans le réservoir d'acide (52).

10. Installation de nettoyage de bioréacteur selon l'une quelconque des revendications 8 à 9, dans laquelle l'unité de commande électronique (44) est conçue pour neutraliser la solution acide aqueuse présente dans le réservoir d'acide (52), pendant que l'unité d'aspiration (72) et/ou l'unité d'alimentation (74) pompe(nt) le liquide depuis le réservoir d'acide (52) vers le premier ou le deuxième capteur de pH (QT60, QT61) et en retour dans le réservoir d'acide (52), à faire en sorte que le doseur de base (88) distribue de la base dans le mélangeur (90) jusqu'à ce qu'une valeur de pH cible neutre (ZpH) soit atteinte dans le réservoir d'acide (52).

11. Procédé d'exploitation d'une installation de nettoyage (1) de bioréacteur, de préférence une installation de nettoyage de bioréacteur (1) selon l'une quelconque des revendications précédentes, comprenant les étapes consistant à:
- déterminer une valeur de qualité d'acide indiquant une qualité d'une solution acide aqueuse présente dans un réservoir d'acide (52) de l'installation de nettoyage (1) de bioréacteur;
- comparer la valeur de qualité d'acide déterminée à une valeur de comparaison d'acide prédéterminée; et,
- en fonction de la comparaison:
- déclencher une neutralisation de la solution acide aqueuse dans le réservoir d'acide (52).

12. Procédé selon la revendication 11, dans lequel la valeur de qualité d'acide est une première valeur de pH, et dans lequel le procédé comprend les étapes consistant à:
- déterminer la première valeur de pH de la solution acide aqueuse présente dans le réservoir d'acide (52);
- comparer la première valeur de pH déterminée à une valeur de pH seuil (SpH) prédéterminée; et dans le cas où la première valeur de pH déterminée est supérieure à la valeur de pH seuil (SpH):
- déclencher la neutralisation de la solution acide aqueuse dans le réservoir d'acide (52).

13. Procédé selon l'une quelconque des revendications 11 à 12, comprenant les étapes consistant à:
- pomper du liquide depuis le réservoir d'acide (52) vers un mélangeur (90) de l'installation de nettoyage (1) de bioréacteur et en retour dans le réservoir d'acide (52); et
- mélanger dans le mélangeur (90) de l'acide et/ou de la base et/ou de l'eau fraîche; et/ou
- déterminer une première valeur de pH en aval du mélangeur (90); et/ou
- déterminer une deuxième valeur de pH en amont du mélangeur (90); et
- en fonction de la première et/ou de la deuxième valeur de pH, mélanger dans le mélangeur (90) de l'acide et/ou de la base afin d'obtenir une valeur de pH cible neutre (ZpH) dans le réservoir d'acide (52); et
- lorsque la valeur de pH cible neutre est atteinte: pomper le liquide depuis le réservoir d'acide (52) dans un réservoir de collecte (50) de l'installation de nettoyage (1) de bioréacteur et/ou dans un canal d'élimination.

14. Procédé selon l'une quelconque des revendications 11 à 13, comprenant les étapes consistant à:
- en fonction de la comparaison: alimenter du liquide dans le bioréacteur (2) depuis le réservoir d'acide (52) afin de nettoyer le bioréacteur (2); et
- aspirer du liquide depuis le bioréacteur (2) et alimenter le liquide aspiré vers le réservoir d'acide (52).

15. Produit de programme d'ordinateur, comprenant des moyens de code qui, lorsqu'ils sont exécutés sur un ordinateur, amènent une installation de nettoyage (1) de bioréacteur à exécuter les étapes selon le procédé selon l'une quelconque des revendications 11 à 14.
